# EUROPEAN PATENT SPECIFICATION

(11) **EP 2 079 476 B1**
(45) Date of publication and mention of the grant of the patent: **21.05.2014**
(21) Application number: 07844402.3
(22) Date of filing: 18.10.2007
(51) Int. Cl.: A61K 31/717, A61P 3/00, A61P 3/04, A61P 3/06, A61P 3/10

(54) **USES OF HYDROXYPROPYL METHYLCELLULOSE FOR PREVENTING OR TREATING METABOLIC SYNDROME**
VERWENDUNG HYDROXYPROPYLMETHYLCELLULOSE ZUR VORBEUGUNG ODER BEHANDLUNG DES STOFFWECHSELSYNDROMS
HYDROXYPROPYL MÉTHYL CELLULOSE UTILISÉS POUR PRÉVENIR OU TRAITER LE SYNDROME MÉTABOLIQUE

(30) Priority: 20.10.2006 US 853388 P
(43) Date of publication of application: 22.07.2009
(73) Proprietor: Dow Global Technologies LLC, Midland, MI 48674 (US); United States Department Of Agriculture, Albany, CA 94710-1105 (US)
(72) Inventor: LYNCH, Stephanie, K., Boonton Township New Jersey 07005 (US); TUROWSKI, Maciej, Midland, MI 48642 (US); YOKOYAMA, Wallace H., Berkeley, CA 94707 (US); HONG, Yun-Jeong, El Cerrito, CA 94530 (US); CONKLIN, Jerry R., Midland, MI 48642 (US); HUNG, Shao-Ching, Midland, MI 48640 (US); YOUNG, Scott A., Midland, MI 48640 (US)
(74) Representative: Beck Greener
(86) International application number: PCT/US2007/081787
(87) International publication number: WO 2008/051794

(56) References cited:
- WO-A-97/07689
- WO-A-03/011258
- WO-A-03/053469
- WO-A-2005/067952
- WO-A-2006/088315
- DE-A1- 10 160 409
- US-A- 5 576 306
- US-A- 5 721 221
- SHAO QIMING ET AL: "Hydroxypropylmethylcellulose (HPMC) prevents insulin resistance in hamsters fed high saturated fat diets through regulation of metabolic genes." ABSTRACTS OF PAPERS AMERICAN CHEMICAL SOCIETY, vol. 229, no. Part 1, March 2005 (2005-03), page U44, XP002474536 & 229TH NATIONAL MEETING OF THE AMERICAN-CHEMICAL-SOCIETY; SAN DIEGO, CA, USA; MARCH 13 -17, 2005 ISSN: 0065-7727
- YOKOYAMA W H ET AL: "Soluble fibers prevent insulin resistance in hamsters fed high saturated fat diets" CEREAL FOODS WORLD, vol. 51, no. 1, January 2006 (2006-01), pages 16-18, XP002474537 ISSN: 0146-6283
- DATABASE BIOSIS [Online] BIOSCIENCES INFORMATION SERVICE, PHILADELPHIA, PA, US; 2004, SHAO QIMING ET AL: "Plasma and Tissue Lipids in Insulin Resistant Hamsters Fed High Fat and Fiber Diets" XP002474538 Database accession no. PREV200400283944 & FASEB JOURNAL, vol. 18, no. 4-5, 2004, pages Abst. 112.8 URL-http://ww, FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638
- DATABASE BIOSIS [Online] BIOSCIENCES INFORMATION SERVICE, PHILADELPHIA, PA, US; March 2003 (2003-03), SHAO QIMING ET AL: "Dietary fiber prevents tissue structure changes and insulin resistance induced by high fat diets in hamsters." XP002474539 Database accession no. PREV200300270536 & FASEB JOURNAL, vol. 17, no. 4-5, March 2003 (2003-03), pages Abstract No. 689.2 URL-http://ww, FASEB MEETING ON EXPERIMENTAL BIOLOGY: TRANSLATING THE GENOME; SAN DIEGO, CA, USA; APRIL 11-15, 2003 ISSN: 0892-6638
- GRUNDY SCOTT M ET AL: "Definition of metabolic syndrome: Report of the National Heart, Lung, and Blood Institute/American Heart Association conference on scientific issues related to definition." CIRCULATION, vol. 109, no. 3, 27 January 2004 (2004-01-27), pages 433-438, XP002473554 ISSN: 0009-7322 cited in the application

## Description

### Field of the Invention

This invention was made under a Cooperative Research And Development Agreement with the US Department of Agriculture, number 58-3K95-5-1072.

This invention relates to a method of preventing or treating metabolic syndrome or a symptom or condition associated with the metabolic syndrome and to a medicament, pharmaceutical composition, food, food ingredient or supplement, or nutraceutical ingredient or supplement useful in such method.

### Background of the Invention

Metabolic syndrome is a complex disease, characterized by the American Heart Association by the following abnormalities: abdominal obesity, atherogenic dyslipidemia, hypertension, insulin resistance with or without glucose intolerance, proinflammatory state and prothrombotic state (Grundy et al., "DEFINITION OF METABOLIC SYNDROME" Circulation, 2004, V109, pages 433-438, Document Number DOI:
10.1 1161/01.CIR.0000111245.75752.C6 available at www.circulationaha.org). It is generally recognized in the art that people with three or more of the above symptoms can be considered to have the metabolic syndrome. The American Heart Association estimates that about 20 to 25 percent of US adults have the metabolic syndrome. People with the metabolic syndrome are at increased risk of a cardiovascular disease, such as coronary heart disease or other diseases related to plaque buildups in artery walls (e.g., stroke and peripheral vascular disease) and/or Type II diabetes. Cardiovascular diseases and type II diabetes belong to the most pervasive diseases in Western populations. Diabetes mellitus is a disease which affects millions people in the United States and, although a heterogeneous disorder, it generally is classified within two major categories, i.e., Type I and Type II diabetes. About 80% of all diabetics in the United States are in the Type II category. This type of diabetes is characterized by both impaired insulin secretion and insulin resistance. The majority of patients are obese
   adults and loss of weight can restore normoglycemia in some cases. However, this type of diabetes can also occur in the non-obese adults and in children. Evidently there is an urgent need to find a method of preventing or treating metabolic syndrome or a symptom or condition associated with the metabolic syndrome.

Since cardiovascular diseases and type II diabetes belong to the most pervasive diseases in Western populations, huge research efforts are not only spent on finding methods of preventing or treating metabolic syndrome, but also on the diagnosis of the symptoms of the metabolic syndrome including microbiological markers and on trying to understand the microbiological processes that influence the various symptoms of the metabolic syndrome.

The above-mentioned article by Grundy et al., "DEFINITION OF METABOLIC SYNDROME", teaches that a proinflammatory state is recognized clinically by elevations of C-reactive protein (CRP). Multiple mechanisms seemingly underlie elevations or CRP. According to the Online Dictionary MidlinePlus Medical Encyclopedia, CRP is a special type of protein produced by the liver that is only present during episodes of acute inflammation. The Medical Encyclopedia indicates that it is not known whether CRP is merely a marker of disease or whether it actually plays a role in causing artherosclerotic disease, but that many consider elevated CRP to be a positive risk factor for coronary artery disease.

The above-mentioned article by Grundy et al., "DEFINITION OF METABOLIC SYNDROME", further teaches that a prothrombotic state is characterized by increased Plasminogen Activator Inhibitor-1 (PAI-1) and fibrinogen. Fibrinogen, an acute-phase reactant like CRP, rises in response to a high-cytokine state. Grundy et al. suggest that prothrombotic state and proinflammatory states may be metabolically interconnected. The Molecular Diagnostics Laboratories MDL, Cincinnati, Ohio, disclose on their Internet Website that PAI-1 is the major inhibitor of fibrinolysis. They suggest that when PAI-1 is high, fibrinolytic activity is depressed, and there is a increased risk for arterial and venous thrombosis. They designate PAI-1 as a significant, independent risk factor for coronary artery disease and ischemic stroke and as a major independent risk factor for venous thrombosis, including osteonecrosis. Juhan-Vague I et al. discuss in Thromb Haemost. 1997 Jul;78(1):656-60 that PAI-1 can be a link between obesity, insulin resistance and cardiovascular disease. Sarah L. Griffiths et al. and David J. Grainger have published in BioEssays 28:629-641, © 2006 Wiley Periodicals, Inc. a hypothesis with the title "Proposal of a novel diabetogenic mechanism involving the serpin PAI-1". In this study they state that elevated levels of the protease inhibitor PAI-1 are well-known molecular markers of the metabolic syndrome. They also present the hypothesis that PAI-1 acts as a causative factor in the development of metabolic syndrome and its clinical sequelae. They suggest that PAI-1, through inhibition of furin, influences insulin resistance, dyslipidemia and low-level inflammation, which corresponds to proinflammatory state and prothrombotic state.

A. Zambon et al. have published in Biochemical Society Transactions (2003) Volume 31, part 5, page 1070 *et seq.* the article "Relevance of hepatic lipase to the metabolism of triacylglycerol-rich lipoproteins". Hepatic lipase (HL) is a glycoprotein that is synthesized and secreted by the liver. HL catalyzes the hydrolysis of triacylglycerols and phospholipids in different lipoproteins.

HL may have pro- as well as anti-atherogenic effects. In the presence of hypertriglyceridaemia or an increased LDL (low density lipoproteins) concentration, the pro-atherogenic effect of high HL may prevail. However, among individuals with low levels of LDL, having high levels of HL may not be atherogenic, but rather anti-atherogenic.

In view of the above-discussed impact of C-reactive protein, of Plasminogen Activator Inhibitor-1, and, depending on the individuals, also of hepatic lipase on one or more symptoms of the metabolic syndrome, it would be desirable to find a method of influencing the level of expression or the concentration of C-reactive protein, of Plasminogen Activator Inhibitor-1; of hepatic lipase or of two or three thereof.

In addition to the impact of C-reactive protein, Plasminogen Activator Inhibitor-1, and hepatic lipase on metabolic syndrome, skilled artisans have also discussed the roles of peroxisome proliferator-activated receptor alpha (PPAR)-alpha and adiponectin in metabolic diseases.

Peroxisome proliferator-activated receptor alpha is a ligand-activated transcriptional factor that belongs to the family of nuclear receptors (van Raalte et al., "Peroxisome proliferator-activated receptor (PPAR)-alpha: a pharmacological target with a promising future" Pharma Research, 2004, Sep,21(9):1531-9). PPAR-alpha regulates the expression of genes involved in fatty acid beta-oxidation and is a major regulator of energy homeostasis. Fibrates are PPAR-alpha agonists and have been used to treat dyslipidemia for several decades because of their triglyceride (TG) lowering and high-density lipoprotein cholesterol (HDL-C) elevating effects. More recent research has demonstrated anti-inflammatory and anti-thrombotic actions of PPAR-alpha agonists in the vessel wall as well. Thus, PPAR-alpha agonists are thought to decrease the progression of atherosclerosis by modulating metabolic risk factors and by their anti-inflammatory actions on the level of the vascular wall. This is confirmed by several clinical studies, in which fibrates have shown to reduce atherosclerotic plaque formation and the event rate of coronary heart disease (CHD), especially in patients suffering from metabolic syndrome. U.S. Patent No. 6,762,171 suggest administering a fatty acid CoA thioester to an individual to inhibit PPAR-alpha in the individual. US Patent Application Publication No. 2004/0115637 suggest compounds, compositions and methods for modulating the expression of PPAR-alpha and for diagnosis and treatment of disease associated with expression of PPAR-alpha. The compositions comprise oligonucleotides.

In view of the correlation between PPAR-alpha and metabolic diseases discussed by the skilled artisans, it would also be'desirable to find a new method of influencing the level of expression or the concentration of PPAR-alpha.

Skilled artisans have suggested adiponectin as a key potential player in metabolic syndrome. Adipocytes express a variety of proteins that function in the homeostatic control of glucose and lipid metabolism. Insulin regulates the translocation and secretion of many of these proteins in response to changes in energy balance. Adipocyte complement-related protein of 30 kDa (Acrp30), now known as adiponectin, is a protein whose secretion from adipocytes is enhanced by insulin stimulation. Adiponectin is an unique and essential adipocytokine that is produced very abundantly in adipocytes and stably present in the plasma at very high concentration (Matsuzawa et al., "Adiponectin and Metabolic Syndrome, Arterioscler Thromb Vasc Biol. 2004;24:29-33). In healthy subjects, adiponectin carries out its roles for preventing development of vascular changes and the impairment of glucose and lipid metabolism, which may be induced by a variety of attacking factors, such as chemical subjects, mechanical stress, or nutritional loading. The above mentioned article by Matsuzawa et al., "Adiponectin and Metabolic Syndrome" suggests that adiponectin may play a key role in the prevention of metabolic syndrome. Hypoadiponectinemia observed in obesity, especially with visceral fat accumulation, is much more frequent than genetic hypoadiponectinemia. Hypoadiponectinemia together with the increase of PAI-1 induced by the accumulation of visceral obesity might be a major background of vascular changes as well as metabolic disorders. Tohru Funahashi, Yuji Matsuzawa and Shinji Kihara, "Adiponectin as a key potential player in metabolic-syndrome", International Congress Series 1262 (2004), Pages 368-371 suggest that hyposecretion of adiponectin may play an important role in the development of obesity-related diseases, particularly atherosclerosis, Diabetes Mellitus, Inflammation and cancer.

Mori Y, Hoshino K, Yokota K, Itoh Y, Tajima N., "Role of hypoadiponectinemia in the metabolic syndrome and its association with post-glucose challenge hyper-free fatty acidemia: a study in prediabetic Japanese males", Endocrine, 2006 Apr;29(2):357-61 suggest that adiponectin is closely associated with the multiple risk factors that go to make up the metabolic syndrome, suggesting a role for hypoadiponectinemia as a surrogate marker for the metabolic syndrome.
In view of the above-discussed impact of adiponectin, specifically of hypoadiponectinemia, on one or more symptoms of the metabolic syndrome, it would be also desirable to find a method of influencing the level of expression or the concentration of adiponectin.

Metabolic syndrome can be prevented or treated by an appropriate, reduced calorie diet consisting of healthy foods (including proper amounts of dietary fiber) and by sufficient exercise (Deen et al., 2004, American Family Physician, V69/12, pp2875-2882). However, many persons suffering from metabolic syndrome are unable to sufficiently change their dietary and exercise habits to prevent the syndrome or to emerge from the syndrome. Thus, there remains a need for a medicament, pharmaceutical composition, food, food ingredient or supplement, or nutraceutical ingredient or supplement to assist persons to prevent metabolic syndrome and to assist persons suffering from metabolic syndrome to emerge from this disease.

Several pharmaceutical compositions, nutraceutical ingredients and dietary supplements have been suggested for treating or preventing individual aspects of the metabolic syndrome.

WO 2004/022074 discloses the use of a composition comprising a non-glucose carbohydrate and soluble fiber or a mixture of pectin and soluble fiber for triggering the secrection of glucagen-like peptide 1, and for controlling metabolic syndrome, diabetes or obesity, or for the promotion of satiety, weight loss or maintenance of the desired body weight. Disclosed non-glucose carbohydrates are galactose, xylose, fructose or mannose. A large variety of soluble fibers is disclosed.

US Patent No. 5,576,306 discloses the use of water-soluble high-viscosity grades cellulose ether compositions for the reduction of serum lipid levels, particularly total serum cholesterol, serum triglycerides, and low-density lipoprotein (LDL) levels and/or attenuation of the postprandial rise of blood glucose levels in animals.

U.S. Patent No. 5,585,366 discloses the use of water-soluble cellulose ethers, such as hydroxypropyl methyl cellulose, for reducing the cholesterol level in mammalian blood.

U.S. Patent No. 6,899,892 discloses the use of water-soluble, non-nutritive, indigestible, non-starch, viscous polysaccharide, such as water-soluble cellulose ethers, for reducing the percentage of body fat and/or the leptin in the bloodstream of the mammal.

U.S. Patent No. 5,721,221 discloses the use of hydroxypropyl methyl cellulose having a viscosity of 50 to 4,000 cps, measured as a 2 weight percent aqueous solution, for reducing total plasma cholesterol levels in a human.

SHAO QIMING ET AL: ABSTRACTS OF PAPERS AMERICAN CHEMICAL SOCIETY, vol. 229, no. Part 1, March 2005, page U44, describes prevention of prevents insulin resistance in hamsters fed high saturated fat diets by hydroxypropyl methylcellulose through regulation of metabolic genes.

YOKOYAMA W H ET AL: CEREAL FOODS WORLD, vol. 51, no. 1, January 2006, pages 16-18, describes prevention of insulin resistance by soluble fibers in hamsters fed high saturated fat diets.

DATABASE BIOSIS [Online] BIOSCIENCES INFORMATION SERVICE, PHILADELPHIA, PA, US; 2004, SHAO QIMING ET AL: Database accession no. PREV200400283944 & FASEB JOURNAL, vol. 18, no. 4-5, 2004, pages Abst. 112.8 describes plasma and tissue lipids in insulin resistant hamsters fed high fat and fiber diets.

DATABASE BIOSIS [Online] BIOSCIENCES INFORMATION SERVICE, PHILADELPHIA, PA, US; March 2003, SHAO QIMING ET AL: Database accession no. PREV200300270536 & FASEB JOURNAL, vol. 17, no. 4-5, March 2003, pages Abstract No. 689.2 discloses that dietary fiber prevents tissue structure changes and insulin resistance induced by high fat diets in hamsters.

WO 97/07689 describes a food formulation comprising an alkyl, or substituted alkyl, edible cellulose compound and an edible polyhydric alcohol. The formulation may be further provided with a time-release coating thereover.

DE 101 60 409 describes an agent for preventing the resorption of fats from the gastro-intestinal tract. Composition (A) for inhibiting resorption of fat from the gastro-intestinal (GI) tract comprises ionic and/or nonionic cellulose ethers (I) that, by forming a gel in the GI tract, completely or partially inhibit cleavage and resorption of fat.

WO 2006/088315 describes a pharmaceutical composition for the treatment and prevention of obesity, diabetes, metabolic syndromes, degenerative diseases and mitochondrial dysfunction-related diseases.

WO 2005/067952 describes a formulation for treating obesity and associated metabolic syndrome. The formulation is a combination of especially selected vegetable extracts: Green tea extract containing Epigallocatehin galate (EGCG), Coleus forskholii extract, Betula alba extract and Guarana or Yerba Maté extract.

WO 03/011258 describes pharmaceutical compositions comprising ketoconazole or a derivative thereof, one or more low melting point lipid excipients and a sufficient quantity of one or more high melting point lipid excipients to provide a liquid and a solid phase within said composition after heating to 35°C for 18 hours and, more particularly, to pharmaceutical compositions comprising ketoconazole or a derivative thereof and a lipid excipient, at least 85 % by weight of said lipid excipient consisting of one or more low melting point lipids and 1-15 % by weight of said lipid excipient consisting of one or more high melting point lipids. Polymeric excipients are also described as is the use of these formulations in the treatment of metabolic syndrome and related conditions.

Co-inventors of the present invention have published at the ACS (American Chemical Society) meeting, San Diego, California, March 15, 2005 that "Hydroxypropylmethylcellulose (HPMC) May Prevent Insulin Resistance in Hamsters Fed High Saturated Fat Diets Through Regulating Metabolic Genes". Syrian hamsters fed a high fat diet similar in fat content to the American diet become insulin resistant (IR). Replacing cellulose in this high fat diet with hydroxypropylmethylcellulose significantly decreases the incidence of insulin resistance. HPMC significantly reduced the glucose infusion rate, fasting plasma insulin, plasma lipids, overall fat distribution in non-adipose tissues, and the cell size of adipose tissues.

The use of water-soluble METHOCEL dietary fiber for slowing fat absorption in a high-fat diet and its potential reduction in the development of insulin resistance, a precursor to Type II diabetes, has subsequently been advertised by The Dow Chemical Company based in the above-mentioned findings of the co-inventors of the present invention.

### Summary of the Invention

While several benefits of water-soluble cellulose derivatives like hydroxypropyl methylcellulose are known, including the benefit for reducing serum lipid levels, which leads to prevention or reduction of atherogenic dyslipidemia, and the benefit for reducing or preventing insulin resistance, it has now been surprisingly found that hydroxypropyl methylcellulose is even useful for preventing or treating three or more of the abnormalities of the metabolic syndrome as defined by the American Heart Association. Accordingly, it has surprisingly been found that hydroxypropyl methylcellulose is even useful for preventing or treating the metabolic syndrome or a symptom or condition associated with the metabolic syndrome in an individual.

In combination with the known effects of water-soluble cellulose derivatives for the prevention or reduction of atherogenic dyslipidemia and the prevention of insulin resistance, it has now been found that hydroxypropyl methylcellulose is useful for preventing or treating the metabolic syndrome or a symptom or condition associated with the metabolic syndrome in an individual. Specifically, it has been found that hydroxypropyl methylcellulose is useful for preventing or treating three or more of the following symptoms of the metabolic syndrome: i) hypertension, ii) proinflammatory or inflammation state, iii) prothrombotic state, iv) atherogenic dyslipidemia and v) insulin resistance with or without glucose intolerance.

More specifically, blood pressure measurements have shown that water-soluble cellulose derivatives are useful for the prevention or treatment of hypertension.

Measurements have also shown that water-soluble cellulose derivatives are useful for influencing the level of expression or the concentration of C-reactive protein (CRP), of Plasminogen Activator Inhibitor-1 (PAI-1), of hepatic lipase (HL) or of two or three thereof in a body tissue. A proinflammatory state, one of the symptoms of the metabolic syndrome, is recognized clinically by elevated concentration or expression level of C-reactive protein (CRP). Elevated concentration or expression level of PAI-1 is an indication of prothrombotic state, another symptom of the metabolic syndrome.

While it is not fully clear yet whether CRP, PAI-1 and HL are only markers of one or more symptoms of metabolic syndrome or actually cause one or more of these symptoms, influencing their level in a body tissue, specifically reducing their level, is an important factor in the prevention or treatment of the metabolic syndrome.

Accordingly, one aspect of the present invention is hydroxypropyl methylcellulose for use in the treatment of metabolic syndrome.

Yet another aspect of the present invention is use of hydroxypropyl methylcellulose for the manufacture of a medicament, pharmaceutical composition, food, food ingredient or supplement, or nutraceutical ingredient or supplement to prevent or treat metabolic syndrome.

Yet another aspect of the present invention is a medicament, pharmaceutical composition, food, food ingredient or supplement, or nutraceutical ingredient or supplement which comprises an effective amount of hydroxypropyl methylcellulose for preventing or treating metabolic syndrome.

### Detailed Description of the Invention

The term "metabolic syndrome" as used herein is characterized by at least three of the following abnormalities: abdominal obesity, atherogenic dyslipidemia, hypertension, insulin resistance with or without glucose intolerance, proinflammatory or inflammation state and prothrombotic state.

The term "a symptom or condition associated with the metabolic syndrome" is defined herein as disclosed in the International Patent Application WO 2004/022074 comprises, but is not restricted to one or more symptoms or conditions selected from hyperglycemia, hyperinsulinaemia, hyperlipidaemia, impaired glucose metabolism, diabetic retinopathy, macular degeneration, cataracts, diabetic nephropathy, glomeruloscerosis, diabetic neuropathy, erectile dysfunction, premenstrual syndrome, vascular restenosis, and/or ulcerative colitis, angina pectoris, myocardial infarction, stroke, skin and/or connective tissue disorders, foot ulcerations, metabolic acidosis, arthritis, osteoporosis and conditions of impaired glucose tolerance. The term "a symptom or condition associated with the metabolic syndrome" also includes cardiovascular diseases or Type II diabetes to the extent that they are associated with the metabolic syndrome.

Abdominal obesity is generally characterized by excess body fat in the region of the abdomen.

Insulin resistance is generally characterized by an impaired ability of the body's insulin to regulate blood glucose metabolism.

Atherogenic dyslipidemia is generally characterized by increased low density lipoprotein [LDL] cholesterol and triglyceride levels and decreased high density lipoprotein [HDL] cholesterol level in blood.

The term hypertension is commonly known as high blood pressure.

The terms "a method of preventing or treating metabolic syndrome or a symptom or condition associated with the metabolic syndrome" and "a method of preventing or treating one or more of the symptoms a) hypertension, b) proinflammatory or inflammation state and c) prothrombotic state" as used herein include any treatment that delays the development of an above-mentioned syndrome or symptom in time or in severity or that reduces the severity of a developing or developed syndrome or symptom.

In the preferred embodiments of the present invention the hydroxypropyl methylcellulose is useful for preventing three or more, more preferably four or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

The term "influencing the level of expression or the concentration of C-reactive protein, of Plasminogen Activator Inhibitor-1 or of hepatic lipase (HL) or of two or three thereof in a body tissue of an individual" means that the body tissue, such as blood, has a different, preferably a lower, level of expression or concentration of CRP and/or PAI-1 and/or HL after the intake of a water-soluble cellulose derivative by an individual, as compared to the level of expression or the concentration of CRP and/or PAI-1 and/or HL after the intake of a non-effective material such as unmodified cellulose itself.

The term "influencing the level of expression of CRP and/or PAI-1 and/or HL" is not limited to the direct regulation of the expression of CRP and/or PAI-1 and/or HL but also includes the indirect influence on CRP and/or PAI-1 and/or HL expression, for example by influencing the conditions or metabolites in a body tissue which lead to a different, preferably lower gene expression.

The term "influencing the level of expression or the concentration of adiponectin in a body tissue of an individual" means that the body tissue, such as blood, has a different, preferably a higher, level of expression or concentration of adiponectin after the intake of a water-soluble cellulose derivative by an individual, as compared to the level of expression or the concentration of adiponectin after the intake of a non-effective material such as unmodified cellulose itself. The term "influencing the level of expression of adiponectin" is not limited to the direct regulation of the expression of adiponectin but also includes the indirect influence on adiponectin expression, for example by influencing the conditions or metabolites in a body tissue which lead to a different, preferably higher gene expression. The term "influencing the level of expression or the concentration perosisome proliferator-activated receptors alpha (PPAR-alpha) in a body tissue of an individual" means that the body tissue, such as blood, has a different, preferably a lower, level of expression or concentration of PPAR-alpha after the intake of a water-soluble cellulose derivative by an individual, as compared to the level of expression or the concentration of PPAR-alpha after the intake of a non-effective material such as unmodified cellulose itself.

The term "influencing the level of expression of PPAR-alpha" is not limited to the direct regulation of the expression of PPAR-alpha but also includes the indirect influence on adiponectin expression, for example by influencing the conditions or metabolites in a body tissue which lead to a different, preferably higher gene expression.

The present invention relates to hydroxypropyl methylcellulose for use in the treatment of individuals, that means any animals including human beings. Preferred individuals are mammals. The term "mammal" refers to any animal classified as a mammal, including human beings, domestic and farm animals, such as cows, nonhuman primates, zoo animals, sports animals, such as horses, or pet animals, such as dogs and cats.

The cellulose derivatives which are useful in the present invention are water-soluble. The term "cellulose derivative" does not include unmodified cellulose itself which tends to be water-insoluble. The term "water-soluble" as used herein means that the cellulose derivative has a solubility in water of at least 2 grams, preferably at least 3 grams, more preferably at least 5 grams in 100 grams of distilled water at 25 °C and I atmosphere.

The preferred cellulose derivative is hydroxypropyl methylcellulose which is classified as a water-soluble cellulose ether by the skilled artisans. The most preferred water-soluble cellulose ethers are hydroxypropyl methylcelluloses with a DS_{methoxyl} of from 0.9 to 2.2, preferably from 1.1 to 2.0, and a MS_{hydroxypropoxyl} of from 0.02 to 2.0, preferably from 0.1 to 1.2. The methoxyl content of methyl cellulose can be determined according to ASTM method D 1347 - 72 (reapproved 1995). The methoxyl and hydroxypropoxyl content of hydroxypropyl methylcellulose can be determined by ASTM method D-2363-79 (reapproved 1989). Methyl celluloses and hydroxypropyl methylcelluloses, such as K100M, K4M, K1M, F220M, F4M and J4M hydroxypropyl methylcellulose are commercially available from The Dow Chemical Company). Combinations of two or more water-soluble cellulose derivatives are also useful.

The water-soluble cellulose derivative generally has a viscosity of from 5 to 2,000,000 cps (= mPa.s), preferably from 50 cps to 200,000 cps, more preferably fromt 75 to 100,000 cps, in particular from 1,000 to 50,000 cps, measured as a two weight percent aqueous solution at 20 degrees Celsius. The viscosity can be measured in a rotational viscometer.

The water-soluble hydroxypropyl methylcellulosecan be administered or consumed in or as a medicament, pharmaceutical composition, food, food ingredient or supplement, or nutraceutical ingredient or supplement. The medicament, pharmaceutical composition, food, food ingredient or supplement, or nutraceutical ingredient or supplement can be solid or liquid. The desired time period of administering the hydroxypropyl methylcellulose can vary depending on the amount of hydroxypropyl methylcellulosederivative consumed, the general health of the individual, the level of activity of the individual and related factors. Since metabolic syndrome or a symptom or condition associated with metabolic syndrome is typically induced by an imbalanced nutrition with a high fat content, it may be advisable to administer or consume the hydroxypropyl methylcelluloseas long as nutrition with a high fat content is consumed. Generally administration of at least 1 to 12 weeks, preferably 3 to 8 weeks is recommended.

It is to be understood that the duration and daily dosages of administration as disclosed herein are general ranges and may vary depending on various factors, such as the specific cellulose derivative, the weight, age and health condition of the individual, and the like. It is advisable to follow the prescriptions or advices of medical doctors or nutrition specialists when consuming the water-soluble cellulose derivatives.

Although water-soluble cellulose derivatives have been used in a variety of foodstuffs to improve certain functional properties, such as emulsification, texture or moisture retention, the amounts used are usually less than 0.5% of the foodstuff. These levels are generally not high enough to have a significant effect on preventing or treating metabolic syndrome or a symptom or condition associated with the metabolic syndrome, and more specifically on preventing or treating hypertension, proinflammatory or inflammation state and prothrombotic state.

According to the present invention hydroxypropyl methylcellulose is preferably used for preparing food, a food ingredient or supplement, or a nutraceutical ingredient or supplement which comprises from 0.5 to 20 weight percent, more preferably from 2 to 15 weight percent, most preferably from 4 to 12 weight percentage of one or more water-soluble cellulose derivatives. The given weight percentages relate to the total amount of the water-soluble cellulose derivatives. The amount administered is preferably in the range of from 1 to 10 percent of the total daily weight of the diet of the individual on a dry weight basis. Preferably, the hydroxypropyl methylcelluloseis administered or consumed in sufficient amounts throughout the day, rather than in a single dose or amount.

Although the hydroxypropyl methylcellulose ispreferably administered in combination with food or as foodstuff, alternatively it can be administered as an aqueous solution or in powder form or as pharmaceutical or nutraceutical compositions. Pharmaceutical or nutraceutical compositions containing water-soluble cellulose derivatives can be administered with an acceptable carrier in a pharmaceutical or nutraceutical unit dosage form. Pharmaceutically acceptable carriers include tableting excipients, gelatin capsules, or carriers such as a polyethylene glycol or a natural gel. Pharmaceutical or nutraceutical unit dosage forms include tablets, capsules, gelatin capsules, pre-measured powders and pre-measured solutions. Hence, the hydroxypropyl methylcellulose preferably is formulated as tablets, granules, capsules and suspensions.

Regardless whether the hydroxypropyl methylcelluloseis administered in solution or in powder form, as a pharmaceutical or nutraceutical composition or is combined with other food ingredients, the amount of administered hydroxypropyl methylcellulose is generally in the range of from 10 to 300 milligrams of hydroxypropyl methylcelluloseper pound of mammal body weight per day. About 2 g to about 30 g, preferably about 3 g to about 15 g of hydroxypropyl methylcellulose is ingested daily by a large mammal such as a human.

While the method of administration or consumption may vary, the hydroxypropyl methylcellulose is preferably ingested by a human as a food ingredient of his or her daily diet. The hydroxypropyl methylcellulose can be combined with a liquid vehicle, such as water, milk, vegetable oil, juice and the like, or with an ingestible solid or semi-solid foodstuff, such as "veggie" burgers, spreads or bakery products. A number of foodstuffs are generally compatible with water-soluble cellulose derivatives. Examples of such foodstuffs are disclosed by M. K. Weibel et al., U.S. Pat. No. 4,923,981. For example, hydroxypropyl methylcellulose may be mixed into foods such as milk shakes, milk shake mixes, breakfast drinks, juices, flavored drinks, flavored drink mixes, yogurts, puddings, ice creams, ice milks, frostings, frozen yogurts, cheesecake fillings, candy bars, including "health bars" such as granola and fruit bars, gums, hard candy, mayonnaise, pastry fillings such as fruit fillings or cream fillings, cereals, breads, stuffing, dressings and instant potato mixes. An effective amount of hydroxypropyl methylcellulose can also be used as a fat-substitute or fat-supplement in salad dressings, frostings, margarines, soups, sauces, gravies, mayonnaises, mustards and other spreads. Therefore, "food ingredients," as the term is used herein, includes those ingredients commonly employed in recipes for the above foodstuffs, including, for example, flour, oatmeal, fruits, milk, eggs, starch, soy protein, sugar, sugar syrups, vegetable oils, butter or emulsifying agents such as lecithin.

The hydroxypropyl methylcellulose can be partially or fully hydrated before it is orally ingested. For example, the hydroxypropyl methylcellulose may be dispersed in a sufficient amount of water, milk, juice, flavored water, hot chocolate, soy milk, cream, or other liquid to make a drink item that can be consumed to administer an effective amount of the water-soluble cellulose derivatives. The hydroxypropyl methylcellulosemay be dispersed in a sufficient amount of water to make a syrupy liquid that is then mixed with one or more food ingredients such as flours, oatmeal, cornmeal, rice, barley, wheat germ, and other cereal products to made a paste or dough, the latter being subsequently treated to create an appealing foodstuff by procedures such as baking, extruding, and the like, to provide edible foodstuffs. Colorings and flavorings may be added as may be appropriate to add to the attractiveness of the foodstuff.

The water-insoluble cellulose derivative can also be administered to domestic and farm animals, such as cows, nonhuman primates, zoo animals, sports animals, such as horses, or pet animals, such as dogs and cats, in a known manner in or as a medicament, pharmaceutical composition, food, food ingredient or supplement, or nutraceutical ingredient or supplement. A preferred way of administration is the incorporation of a water-insoluble cellulose derivative in the pet feed or other animal feed. The hydroxypropyl methylcelluloseis optionally used in combination with water-soluble or water-insoluble naturally occurring polymers or derivatives thereof, such as gum arabic, xanthan gum or derivatives thereof, gum karaya, gum tragacanth, gum ghatti, guar gum or derivatives thereof, exudate gums, seaweed gums, seed gums, microbial gums, carrageenan, dextran, gelatin, alginates, pectins, starches or derivatives thereof, chitosans or other polysaccharides, preferably beta-glucans, galactomannans, hemicelluloses, psyllium, guar, xanthan, microcrystalline cellulose, amorphous cellulose or chitosan.

In some embodiments of the present invention it is particularly beneficial to use or administer hydroxypropyl methylcellulose in combination with a water-insoluble cellulose derivative. Useful amounts of combinations of one or more water-soluble cellulose derivatives and one or more water-insoluble cellulose derivatives and useful ways for administration, consumption or inclusion of such combinations in a pharmaceutical composition, food, food ingredient or supplement, or nutraceutical ingredient or supplement are generally the same as those described above for the water-soluble cellulose derivatives alone.

Useful water-insoluble cellulose derivatives have a solubility in water of less than 2 grams, preferably less than 1 gram, in 100 grams of distilled water at 25 °C and 1 atmosphere. Preferred water-insoluble cellulose derivatives are water-insoluble cellulose ethers, particularly ethyl cellulose, propyl cellulose or butyl cellulose. Other useful water-insoluble cellulose derivatives are cellulose derivatives which have been chemically, preferably hydrophobically, modified to provide water insolubility. Chemical modification can be achieved with hydrophobic long chain branched or non-branched alkyl, arylalkyl or alkylaryl groups. "Long chain" typically means at least 5, more typically at least 10, particulary at least 12 carbon atoms. Other types of water-insoluble cellulose are crosslinked cellulose, when various crosslinking agents are used. Chemically modified, including the hydrophobically modified, water-insoluble cellulose derivatives are known in the art. They are useful provided that they have a solubility in water of less than 2 grams, preferably less than 1 gram, in 100 grams of distilled water at 25 °C and 1 atmosphere. The most preferred cellulose derivative is ethyl cellulose. The ethyl cellulose preferably has an ethoxyl substitution of from 40 to 55 percent, more preferably from 43 to 53 percent, most preferably from 44 to 51 percent. The percent ethoxyl substitution is based on the weight of the substituted product and determined according to a Zeisel gas chromatographic technique as described in ASTM D4794-94(2003). The molecular weight of the ethyl cellulose is expressed as the viscosity of a 5 weight percent solution of the ethyl cellulose measured at 25°C in a mixture of 80 volume percent toluene and 20 volume percent ethanol. The ethyl cellulose concentration is based on the total weight of toluene, ethanol and ethyl cellulose. The viscosity is measured using Ubbelohde tubes as outlined in ASTM D914-00 and as further described in ASTM D446-04, which is referenced in ASTM D914-00. The ethyl cellulose generally has a viscosity of up to 400 mPa·s, preferably up to 300 mPa·s, more preferably up to 100 mPa·s, measured as a 5 weight percent solution at 25°C in a mixture of 80 volume percent toluene and 20 volume percent ethanol. The preferred ethyl celluloses are premium grades ETHOCEL ethyl cellulose which are commercially available from The Dow Chemical Company of Midland, Michigan.

The present invention is further illustrated by the following examples which are not to be construed to limit the scope of the invention. Unless otherwise mentioned, all parts and percentages are by weight.

### EXAMPLES

### Example 1

An animal study was conducted with male golden Syrian hamsters with a starting body weight of 70-90 grams (Sasco strain, Charles River, Wilmington, MA). The animal study was approved by the Animal Care and Use Committee, Western Regional Research Center, USDA, Albany, CA. The hamsters were divided into two groups. One of the groups was called "treatment group" and was fed a high-fat treatment diet and water ad libitum, while the other group was called "control group" and was fed high-fat control diet and water ad libitum. Both groups counted 10 hamsters each. These groups were fed for a period of eight consecutive weeks.

A water-soluble cellulose ether was present at 5 weight percent level in the treatment diet. It was mixed with the powdered components of the diet. The water-soluble cellulose ether was hydroxypropyl methylcellulose (HPMC). The HPMC had a methoxyl content of 19-24 percent, a hydroxypropoxyl content of 7-12 percent and a viscosity of about 100,000 mPa·s, measured as a 2 wt.% aqueous solution at 20°C, and is commercially available from The Dow Chemical Company under the Trademark METHOCEL K100M hypromellose. 1000 g of either of the complete high-fat treatment diets contained 150 g of butter fat, 50 g of corn oil, 200 g of casein, 499 g of corn starch, 3 g ofDL methionine, 3 g of choline bitartrate, 35 g of a mineral mixture, 10 g of a vitamin mixture and 50 g of METHOCEL K100M hypromellose.

The control diet had exactly the same composition as the treatment diet, with the only exception that the water-soluble cellulose ether was replaced by same amount of microcrystalline cellulose (MCC), mixed into powdered components of the diet during the control diet preparation.

After the hamsters had been fed the diets for eight consecutive weeks, the livers were taken out from four or more animals of the Treatment Group and four or more animals of the Control Group on a random basis. The sacrified hamsters of the Treatment Group are designated in Table 1 below as HF-HPMC-1, HF-HPMC-2, HF-HPMC-3, HF-HPMC-4, HF-HPMC-5, HF-HPMC-6 and HF-HPMC-7. The hamsters of the Control Group are designated in Table 1 below as HF-Control-1 and HF-Control-2, HF-Control-3 and HF-Control-4.

Messenger ribonucleic acid (mRNA) was extracted from these livers of these hamsters. Total mRNA was extracted, purified, and reverse transcribed according to Bartley and Ishida (2002). The teaching of Bartley, G.E. and Ishida, B.K. (2002) Digital Fruit Ripening: Data Mining in the TIGR Tomato Gene Index. Plant Mol. Biol. Rep. 20: 115-130.

cDNAs resulting from reverse transcription of the above total mRNAs were diluted 10 fold and 1 microliter aliquots were used in real-time PCR reactions with specific primers for the genes having a length of 20-24 bases as described further below with SYBR Green Supermix (BIO-RAD) according to the manufacturer's protocols with the following changes: 1. Reactions were performed in 25-microliter total volume in triplicate reactions 2. An MX3000P (Stratagene) instrument was used to perform the PCR. PCR conditions were 5 min at 95°C followed by 40 cycles of incubation at 94 °C x 15 s, 55 to 60 °C x 1 min and 72 °C x 30 s.

The following primers were used:

| | |
|---|---|
| CRP: | CGTGTTGTCATTATGTAGGTCTTA (forward), |
| | GTAGCTTTATTGACTCATGGACC (reverse); |
| | |
| PAI-1: | TTCACAAGTCTTTCCGACCAA (forward), |
| | GGGGGCCATGCGGGCTGAGA (reverse); |
| | |
| HL: | AAGAGAATTCCCATCACCCTG (forward), |
| | CTGTTTTCCCACTTGAACTTGA (reverse); |
| | |
| Actin: | ACGTCGACATCCGCAAAGACCTC (forward), |
| | GATCTCCTTCTGCATCCGGTCA (reverse). |

Primer efficiencies were determined using dilution curves of cDNA. Relative quantitation was performed by normalization to the actin transcript as in Livak, K.J. and Schmittgen, T.D. (2001). The teaching of Livak, K.J. and Schmittgen, T.D. (2001), Analysis of relative gene expression data using real-time quantitative PCR and the 2-ΔΔCT Method. Methods. 25: 402-408. Negative controls to determine the extent of DNA contamination were carried out with identical concentrations of total mRNAs (samples after purification) without reverse transcription. A negative control was run for some of the primer sets. In each case the no-reverse transcription control signal was achieved after 5 or more cycles than the samples that were transcribed.

The C-reactive protein (CRP), Plasminogen Activator Inhibitor-1 (PAI-1) and hepatic lipase (HL) gene expression of the hamster HF-HPMC-1 was compared with the CRP, PAI and HL gene expression of the hamsters HF-Control- and HF-Control-2. The ratios for the gene expressions HF-HPMC-1/ HF-Control-1 and HF-HPMC-1/ HF-Control-2 are listed in Table 1 below. The ratios for the CRP, PAI and HL gene expression of the other pairs of hamsters were determined as listed in Table 1 below.

The results are listed in Table 1 below. The values in Table 1 for each animal pair and each gene are an average of triplicate measurements. The mean and standard error of the mean (SEM) values are given. It is understood that the numbers expressed in the Table 1 are relative to control, i.e. if the number is lower than 1 then the expression of a particular gene is lower in the hamsters from the treatment group than in the hamsters from the control group, and vice versa.

**Table 1**

| **Animal pairs, ratio of gene expression after 8 weeks feeding** | **CRP** | **PAI-1** | **HL** |
|---|---|---|---|
| -HF-HPMC-1/ HF-Control-1 | 3.1±1.5 * | 0.73±0.48 | 0.84±0.13 |
| HF-HPMC-1/ HF-Control-2 | 2.5±1.3* | 0.65±0.34 | 0.87±0.06 |
| HF-HPMC-2/ HF-Control-1 | 1.1±0.27 | 0.57±0.04 | 0.77±0.19 |
| HF-HPMC-2/ HF-Control-2 | 0.81±0.13 | 0.62±0.16 | 0.79±0.03 |
| HF-HPMC-3/ HF-Control-3 | 0.61±0.2 | 1.1±0.35 | 0.58±0.17 |
| HF-HPMC-3/ HF-Control-4 | 1.1±0.3 | 0.67±0.2 | 0.70±0.05 |
| HF-HPMC-4/ HF-Control-3 | 0.5±0.06 | 1.2±0.36 | 0.74±0.33 |
| HF-HPMC-4/ HF-Control-4 | 0.97±0.16 | 0.72±0.09 | 0.88±0.23 |
| HF-HPMC-5/ HF-Control-3 | 0.65±0.02 | 1.02±0.1 | Not measured |
| HF-HPMC-5/ HF-Control-4 | 1.14±0.1 | 0.79±0.17 | Not measured |
| HF-HPMC-6/ HF-Control-3 | 0.69±0.06 | 1.63±0.2 | Not measured |
| HF-HPMC-6/ HF-Control-4 | 1.22±0.04 | 1.25±0.14 | Not measured |
| HF-HPMC-7/ HF-Control-3 | 0.52±0.08 | 0.93±0.37 | Not measured |
| HF-HPMC-7/ HF-Control-4 | 0.92±0.17 | 0.69±0.2 | Not measured |
| **Mean** | **0.85** | **0.90** | **0.77** |
| **SEM (Standard Error of Mean)** | **0.07** | **0.08** | **0.04** |

| | | | |
|---|---|---|---|
| * Eliminated for calculating Mean and SEM based on "Standard Practice for Dealing With Outlying Observations" ASTM E 178 - 80. A statistical outlier analysis was done using the Grubb's analysis [Grubbs, Frank (February 1969), Procedures for Detecting Outlying Observations in Samples, Technometrics, Vol. 11, No. 1, pp. 1-21 and http://www.itl.nist.gov/div898/handbook/eda/section3/eda35h.htm]. | | | |

While the data show some variation within the same group of animals, this is to be expected since the results are obtained on biological, living systems. Nevertheless, the data show a clear trend. The CRP, PAI-1 and HL gene expressions are generally lower in the animals of the Treatment Group that were fed a diet containing water-soluble hydroxypropyl methylcellulose than in the animals of the Control Group that were fed a diet comprising microcrystalline cellulose instead of a water-soluble cellulose derivative.

The results in Table 1 above illustrate the usefulness of a water-soluble cellulose derivative, such as hydroxypropyl methylcellulose for preventing or treating proinflammatory or inflammation state and prothrombotic state, two of the symptoms of the metabolic syndrome.

### Example 2

Male Syrian golden hamsters of the same type and starting body weight as in Example 1 were divided into five groups. The groups counted 10 hamsters each. These groups were fed for a period of eight consecutive weeks.

To prepare the diets of the Treatment groups, a water-soluble cellulose ether was mixed with the powdered components of the diet. The water-soluble cellulose ether was hydroxypropyl methylcellulose (HPMC). The HPMC had a methoxyl content of 19-24 percent, a hydroxypropoxyl content of 7-12 percent and a viscosity of about 100,000 mPa·s, measured as a 2 wt.% aqueous solution at 20°C, and is commercially available from The Dow Chemical Company under the Trademark METHOCEL K100M hypromellose.

Treatment Group 1: High Fat Diet, 5 weight percent HPMC.

This treatment group was fed a high-fat treatment diet and water ad libitum. 1000 g of the high-fat treatment diet contained 150 g of butter fat, 50 g of corn oil, 200 g of casein, 499 g of corn starch, 3 g of DL methionine, 3 g of choline bitartrate, 35 g of a mineral mixture, 10 g of a vitamin mixture and 50 g of METHOCEL K100M hypromellose.

Treatment Group 2: High Fat Diet, 2.5 weight percent HPMC.

The high-fat treatment diet for Treatment Group 2 was the same as the diet for Treatment Group 1, except that it contained 25 g METHOCEL K100M hypromellose and 25 g of microcrystalline cellulose (MCC) instead of 50 g of METHOCEL K100M hypromellose.

### Control Group 1:

The high-fat treatment diet for Control Group 1 was the same as the diet for Treatment Group 1, except that it contained 50 g of microcrystalline cellulose (MCC) instead of 50 g of METHOCEL K100M hypromellose.

### Control Group 2:

The high-fat treatment diet for Control Group 1 was the same as the diet for Treatment Group 1, except that it contained 50 g of Psyllium instead of 50 g of METHOCEL K100M hypromellose. Psyllium has been reported to be effective in reducing cholesterol and blood glucose response.

### Low Fat Control Group

The low-fat treatment diet contained most of the same ingredients at the same proportions as Control Group 1, except that it did not contain 150 g of butterfat and it only contained 50 g of corn oil, thus it had 5% fat in general. The weight of butterfat (150 g) was replaced by the same amount of corn starch. Similar to Control Group 1, the Low Fat Control Group contained 50 g of microcrystalline cellulose (MCC) instead of 50 g of METHOCEL K100M hypromellose.

The systolic blood pressure of the hamsters was measured after having fed the hamsters for eight weeks with the above-described experimental diets. The results are listed in Table 2 below. Each value references an average of 13-14 animals with 3-5 repetitive measurements per animal. The values of the 3-5 repetitive measurements for each animal were subjected to a statistical outlier analysis using the Grubb's analysis [Grubbs, Frank (February 1969), Procedures for Detecting Outlying Observations in Samples, Technometrics, Vol. 11, No. 1, pp. 1-21. and http://www.itl.nist.gov/div898/handbook/eda/section3/eda35h.htm].

**Table 2**

| | **Change in absolute numbers** | | | |
|---|---|---|---|---|
| **Diet** | **Mean BP Systolic [mm Hg] and Standard Error of Mean (SEM)** | | | |
| | **Week 0** | **Week 2** | **Week 4** | **Week 8** |
| **Treatment Group 1, HPMC 5%** | 101.4±3.7 | 96.7±3.3 | 93.7±3.3 | 93.2±5.5 |
| **Treatment Group 2, HPMC 2.5 %** | 101.1±3.8 | 93.8±3.3 | 93.7±3.6 | 91.7±3.8 |
| | | | | |
| **Control Group 1, High Fat diet** | 100.1±6.0 | 96.2±3.6 | 100.6±4.9 | 101.9±4.6 |
| **Control Group 2, Psyllium 5%** | 98.6±4.7 | 95.9±4.4 | 102.4±4.6 | 99.8±8.6 |
| | | | | |
| **Control Group 3, low fat diet** | 106.9±6.1 | 100.7±4.1 | 100.2±4.1 | 96.0±8.4 |

Since the blood pressures vary from one animal to the next, not the absolute numbers should be considered to evaluate the effectiveness of a diet on the blood pressure, but its change over time. The percent change of the blood pressure over time, using the blood pressure in Week 0 as the baseline, is listed in Table 3 below.

**Table 3**

| **Diet** | **Percent Change** | | | |
|---|---|---|---|---|
| | **Week 0** | **Week 2** | **Week 4** | **Week 8** |
| **Treatment Group 1, HPMC 5%** | 0.0 | -4.9 | -8.2 | -8.8 |
| **Treatment Group 2, HPMC 2.5 %** | 0.0 | -7.8 | -7.9 | -10.3 |
| | | | | |
| **Control Group 1, High Fat diet** | 0.0 | -4.1 | 0.5 | 1.7 |
| **Control Group 2, Psyllium 5%** | 0.0 | -2.8 | 3.7 | 1.2 |
| | | | | |
| **Control Group 3, low fat diet** | 0.0 | -6.2 | -6.6 | -11.4 |

The results in Tables 2 and 3 above illustrate the usefulness of a water-soluble cellulose derivative, such as hydroxypropyl methylcellulose for preventing or treating hypertension, one of the symptoms of the metabolic syndrome. The blood pressure of the animals treated according to the present invention decreased, although they were fed the same high fat diet as the animals of the Control Groups 1 and 2. The decrease in blood pressure was even comparable to the decrease in blood pressure in the Control Group 3 which was fed a low fat diet. In contrast thereto, the blood pressure of the animals that were fed a high fat diet with or without psyllium slightly increased.

### Example 3

An animal study was conducted with male golden Syrian hamsters with a starting body weight of 50-60 grams (LVG strain, Charles River, Wilmington, MA) in each of the diets specified below. The animal study was approved by the Animal Care and Use Committee, Western Regional Research Center, USDA, Albany, CA.

The male Syrian golden hamsters were divided into six groups. Five groups were called "treatment group" and were fed diets containing HPC-L, K100M, HEC, MEC1, and MEC2. One group was called "control group" and was fed a diet consisting of microcrystalline cellulose (MCC). Each group consisted of approximately 10 hamsters each. These groups were fed for a period of three consecutive weeks.

### Treatment Group 1: K100M

This treatment group was fed a METHOCEL K100M hypromellose treatment diet. 1000 g of the METHOCEL K100M hypromellose treatment diet contained 80 g of butter fat, 100 g of corn oil, and 20 g of fish oil and 1 g of cholesterol, 200 g of casein, 498 g of corn starch, 3 g of DL methionine, 3 g of choline bitartrate, 35 g of a mineral mixture, 10 g of a vitamin mixture and 50 g of METHOCEL K 100M hypromellose.

### Treatment Group 2: MEC1 (comparative)

The MEC1 diet for Treatment Group 2 was the same as the diet for Treatment Group 1, except that it contained 50 g of methyl ethylcelluose instead of 50 g of METHOCEL K100M hypromellose. MEC1 had a Brookfield viscosity of 11,300 cP, measured as a 2 weight percent aqueous solution, a methoxyl substitution of 4.1 percent and an ethoxyl substitution of 18.7 percent.

### Treatment Group 3: MEC2 (comparative)

The MEC2 diet for Treatment Group 3 was the same as the diet for Treatment Group 1, except that it contained 50 g of methyl ethylcellulose instead of 50 g of METHOCEL K100M hypromellose. MEC2 had a Brookfield viscosity of 400 cP, measured as a 2 weight percent aqueous solution, a methoxyl substitution of 4.6 percent methoxyl and an ethoxyl substitution of 18.4 percent.

### Treatment Group 4: HPC-L (comparative)

The HPC-L diet for Treatment Group 4 was the same as the diet for Treatment Group 1, except that it contained 50 g of hydroxypropyl cellulose, designated as HPC-L instead of 50 g of METHOCEL K100M hypromellose. HPC-L had a viscosity of 1800-3000 cP (1% solution, 25°C, by Brookfield @ 30 RPM) and was commercially available from Aldrich as hydroxypropylcellulose and had the lot No. 08031ED.

### Treatment Group 5: HEC (comparative)

The HEC diet for Treatment Group 5 was the same as the diet for Treatment Group 1, except that it contained 50 g of hydroxyethyl cellulose, designated as HEC, instead of 50 g of METHOCEL K100M hypromellose. HEC had a viscosity 17,000 cp and was commercially available from Hercules under the Trademark Natrosol and had the lot No. T-0439.

### Control Group: MCC

The control diet had exactly the same composition as the diet for the Treatment Group 1, with the only exception that METHOCEL K100M hypromellose was replaced by same amount of microcrystalline cellulose (MCC), mixed into powdered components of diet during the control diet preparation.

After the hamsters had been fed the diets for three consecutive weeks, plasma was obtained and the livers were taken out from both the treatment groups and control group. The sacrificed hamsters of the treatment group are designated in Table 4 below as HPC-L, K100M, HEC, MEC1, and MEC2. The sacrificed hamsters of the control group are designated in Table 4 below as MCC.

### Quantitative RT-PCR Analysis PPARα and PAI-1 in hamster livers

The gene expressions for Plasminogen Activator Inhibitor-1 (PAI-1) and perosisome proliferator-activated receptors alpha (PPARα), was determined by mRNA extraction and analysis as described in Example 1. Total mRNA was extracted, purified, and reverse transcribed according to Bartley and Ishida (2002), as described in Example 1. The following primers were used:
PPARα: CTCCACCTGCAGAGCAACCA (forward),
   CGTCAGACTCGGTCTTCTTGAT (reverse);
PAI-1: TTCACAAGTCTTTCCGACCAA (forward),
   GGGGGCCATGCGGGCTGAGA (reverse).

The PPARα and PAI-1 gene expression of the hamsters that had been fed the diets HPC-L, K100M, HEC, MEC1, and MEC2 was compared with PPARα and PAI-1 gene expression of the hamsters of the control MCC group. The ratios for the gene expression are listed in Table 4 below. The mean and standard error of the mean (SEM) values are given. It is understood that the numbers expressed in the Table 4 are relative to control, i.e. if the number is lower than 1 then the expression of a particular gene is lower in hamsters from the treatment group than in the hamsters from the control group, and vice versa.

**Table 4**

| **Diet** | **PPARα Mean (SEM)** | **PAI-1 Mean (SEM)** |
|---|---|---|
| HPC-L (comparative) | 0.64 (0.06) | 0.82 (0.13) |
| K100M | 1.06 (0.08) | 0.44 (0.05) |
| HEC (comparative) | 0.65 (0.15) | 0.77 (0.12) |
| MEC1 (comparative) | 0.69 (0.10) | 0.95 (0.14) |
| MEC2 (comparative) | 0.95 (0.11) | 0.83 (0.09) |

While the data show some variation within the same group of animals, this is to be expected since the results are obtained on biological, living systems. Nevertheless, the data show some clear trends. The PPARα and PAI-1 gene expressions are generally lower in the animals of the Treatment Groups that were fed a diet containing a water-soluble cellulose derivative than in the animals of the Control Group that were fed a diet comprising microcrystalline cellulose instead of a water-soluble cellulose derivative.

The results in Table 4 above illustrate the usefulness of a water-soluble cellulose derivative, such as hydroxypropyl methylcellulose for preventing or treating proinflammatory or inflammation state and prothrombotic state, two of the symptoms of the metabolic syndrome.

### Example 4

An animal study was conducted with male golden Syrian hamsters with a starting body weight of 50-60 grams (LVG strain, Charles River, Wilmington, MA) in each of the diets specified below. The animal study was approved by the Animal Care and Use Committee, Western Regional Research Center, USDA, Albany, CA.

The male Syrian golden hamsters were divided into two groups. One group was called "treatment group" and was fed a diet containing K100M. One group was called "control group" and was fed a diet containing microcrystalline cellulose (MCC). Each group consisted of approximately 10 hamsters each. These groups were fed for a period of sixteen consecutive weeks.

### Treatment Group 1: K100M

This treatment group was fed a K100M treatment diet. 1000 g of the METHOCEL K100M hypromellose treatment diet contained 150 g of butter fat, 50 g of corn oil, 200 g of casein, 499 g of corn starch, 3 g of DL methionine, 3 g of choline bitartrate, 35 g of a mineral mixture, 10 g of a vitamin mixture and 50 g of METHOCEL K100M hypromellose.

### Control Group: MCC

The control diet had exactly the same composition as the treatment diet, with the only exception that METHOCEL K100M hypromellose was replaced by the same amount of microcrystalline cellulose (MCC), mixed into powdered components of diet during the control diet preparation.

### Analysis of PAI-1 in Hamster Plasma

Hamster EDTA plasma samples were assayed for PAI-1 activity based on the inhibition of the plasminogen activator (urokinase (uPA) or tissue plasminogen activator (tPA)) activity of the synthetic chromogenic substrate method.

Plasma samples were assayed directly using the colorimetric assay of PAI-1 based on the procedures provided with the assay kit, STACHROM PAI, Diagnostica Stago (Parsippany, NJ). A protocol for microplate format was used. After reconstituting all reagents, 25 µL of plasma or PAI calibrator and 100 µL of Reagent 1 (uPA) were added to the designated wells. The plate was incubated in the pre-warmed plate reader at 37 °C for 4 minutes. This step initiated the binding between PAI-1 and uPA. For measuring the residual uPA activity after PAI-1 inhibition, 100 µL of Reagent 2 (plasminogen) was added to each well and the reaction mixture was incubated at 37 °C for 4 minutes. Plasmin was generated as a result of the reaction, and the amidolytic activity of plasmin was determined by the reaction kinetics upon addition of 100 µL of prewarmed substrate (Reagent 3) at 37 °C. The absorbance at 405 nm was measured at 15 seconds and 45 seconds after the addition substrate. Because the assay was performed in the kinetic mode, the reagents should be added quickly and the precise time of each reagent addition should be noted. The PAI-1 level was determined based on the standard curve generated by plotting Δabsorbance value of the two time point versus the calibrator activity level provided with specific lot.

### Analysis of Adiponectin in Hamster Plasma

Hamster EDTA plasma samples were assayed for adiponectin based on a double-antibody sandwich enzyme immunoassay technique.

Plasma samples were diluted prior to the start of the assay with reagent buffers from the Adiponectin ELISA Kit, B-Bridge International, Inc. (Mountain View, CA). After reconstituting all reagents, 100 µL of serially diluted adiponectin standards and diluted plasma sample were added to the appropriate number of antibody-coated wells. Adiponectin in the sample binds to the primary anti-adiponectin polycolonal antibody immobilized in the well (1^{st} reaction). The plates were incubated at 22-28°C for 60 minutes. Following incubation each well was washed three times with the wash buffer. After washing, 100 µL of biotinylated secondary anti-adiponectin polyclonal antibody was added to each well and allowed to incubate at 22-28°C for 60 minutes (2^{nd} reaction). The biotinylated secondary rabbit anti-adiponectin polyclonal antibody binds to the adiponectin trapped in the well in the 1^{st} Reaction. Following incubation each well was washed three times with the wash buffer. After washing, a conjugate of horseradish peroxidase (HRP) and streptavidin was added to each well and allowed to incubate at 22-28°C for 60 minutes (Reaction 3). The HRP-conjugated streptavidin recognizes and binds to the biotinylated rabbit anti-adiponectin antibody trapped in the well in the 2^{nd} Reaction. After washing, the colorimetric substrate for the enzyme is added to all wells and incubated. The color development is terminated by the addition of a stop solution. The absorbance of each well was measured at 450 nm with a Synergy™ HT Multi-Detection Microplate Reader.

After the hamster plasma was obtained from the different diets the plasma was analyzed for both PAI-1 and adiponectin. The results are listed in Table 5.

**Table 5**

| Diet | [PAI] | Ratio | [Adiponectin] | Ratio |
|---|---|---|---|---|
| K100M | 4.9±0.3 | 0.97 | 11.3±1.8 | 1.21 |
| MCC | 5.1±0.4 | - | 9.4±1.4 | - |

| | | | | |
|---|---|---|---|---|
| *mean ± standard deviation | | | | |

The PAI-1 level in hamster plasma was measured by an enzymatic method, which has been used to measure bioactive PAI-1 protein in rat (cell cultures or animal). The measured PAI-1 levels in hamster plasma samples of this study are expressed as amidolytic units (AU) per mL. The PAI-1 protein levels are lower in the animals of the Treatment Group that were fed a diet containing hydroxypropyl methylcellulose than in the animals of the Control Group that were fed a diet comprising microcrystalline cellulose. The levels of adiponectin were higher for hamsters fed with hydroxypropyl methylcellulose diet than the animals of the Control Group that were fed a diet comprising microcrystalline cellulose.

Collectively, the decrease in PAI-1 and the increase in adiponectin protein expression is an important factor in the prevention or treatment of metabolic syndrome.

### Example 5

An animal study was conducted with male golden Syrian hamsters with a starting body weight of 50-60 grams (LVG strain, Charles River, Wilmington, MA) in each of the diets specified below. The animal study was approved by the Animal Care and Use Committee, Western Regional Research Center, USDA, Albany, CA.

The male Syrian golden hamsters were divided into two groups. One group was called "treatment group" and was fed a diet containing hydroxypropyl methylcellulose K100M. One group was called "control group" and was fed a diet comprising of microcrystalline cellulose (MCC). Each group consisted of approximately 10 hamsters each. These groups were fed for a period of three consecutive weeks.

### Treatment Group 1: K100M

This treatment group was fed a K100M treatment diet. 1000 g of the METHOCEL K100M hypromellose treatment diet contained 80 g of butter fat, 100 g of corn oil, and 20 g of fish oil and 1 g of cholesterol, 200 g of casein, 498 g of corn starch, 3 g of DL methionine, 3 g of choline bitartrate, 35 g of a mineral mixture, 10 g of a vitamin mixture and 50 g of METHOCEL K100M hypromellose.

### Control Group: MCC

The control diet had exactly the same composition as the treatment diet, with the only exception that the hydroxypropyl methylcellulose derivative was replaced by same amount of microcrystalline cellulose (MCC), mixed into powdered components of diet during the control diet preparation.

### Analysis of Adiponectin in Hamster Plasma

Hamster EDTA plasma samples were assayed for adiponectin based on a double-antibody sandwich enzyme immunoassay technique. The adiponectin assay was conducted in a similar manner as described in Example 4.

### Analysis of PAI-1 in Hamster Plasma

Hamster EDTA plasma samples were assayed for Plasminogen Activator Inhibitor-1 (PAI-1) activity based on the inhibition of the plasminogen activator (urokinase (uPA) or tissue plasminogen activator (tPA)) activity of the synthetic chromogenic substrate method. The PAI-1 assay was conducted in a similar manner as described in Example 4.

After the hamster plasma was obtained from the different diets the plasma was analyzed for PAI-1 and adiponectin. The results are listed in Table 6.

**Table 6**

| **Diet** | **[PAI]** | **Ratio** | **[Adiponectin]** | **Ratio** |
|---|---|---|---|---|
| K100M | 4.1±1.3 | 0.95 | 10.3±2.3 | 1.54 |
| MCC | 4.3±0.6 | - | 6.7±1.6 | - |

| | | | | |
|---|---|---|---|---|
| *mean ± standard deviation | | | | |

As previously described, the PAI-1 level in hamster plasma was measured by an enzymatic method, which has been used to measure bioactive PAI-1 protein in rat (cell cultures or animal). The measured PAI-1 levels in hamster plasma samples of this study are expressed as amidolytic units (AU) per mL. The data show some variation; this is to be expected since the results are obtained on biological, living systems. The data from hamster plasma for this feed study shows a trend that is similar to the data from liver for the gene expression and activity assays for PAI-1 in Example 3.

Compared to the control (MCC) diet, the data on Plasma adiponectin concentrations in hamster samples show a clear trend. The levels of adiponectin were significantly higher for hamsters fed with K100M diet than the animals of the Control Group that were fed a diet comprising microcrystalline cellulose.
Collectively, the decrease in PAI-1 and the increase in adiponectin protein expression is an important factor in the prevention or treatment of metabolic syndrome.

### Example 6

An animal study was conducted with male golden Syrian hamsters with a starting body weight of 50-60 grams (LVG strain, Charles River, Wilmington, MA) in each of the diets specified below. The animal study was approved by the Animal Care and Use Committee, Western Regional Research Center, USDA, Albany, CA.

The male Syrian golden hamsters were divided into five groups. Four groups were called "treatment group" and were fed a diet containing hydroxypropyl methylcellulose. One group was called "control group" and was fed a diet containing microcrystalline cellulose (MCC). Each group consisted of approximately 10 hamsters each. These groups were fed for a period of three consecutive weeks.

### Treatment Group 1: J1M

This treatment group was fed a J1M hydroxypropyl methylcellulose treatment diet. 1000 g of the J1M treatment diet contained 80 g of butter fat, 100 g of corn oil, and 20 g of fish oil and 1 g of cholesterol, 200 g of casein, 498 g of corn starch, 3 g of DL methionine, 3 g of choline bitartrate, 35 g of a mineral mixture, 10 g of a vitamin mixture and 50 g of J1M hydroxypropyl methylcellulose. J1M hydroxypropyl methylcellulose has a methoxyl content of about 18 percent, a hydroxypropoxyl content of about 27 percent and a Brookfield viscosity of about 1,000 mPa·s, measured as a 2 wt.% aqueous solution at 20°C, and is commercially available from The Dow Chemical Company under the Trademark METHOCEL J1M hypromellose.

### Treatment Group 2: J75M

The J75M diet for Treatment Group 2 was the same as the diet for Treatment Group 1, except that it contained 50 g of hydroxypropyl methylcellulose J75M. J1M hydroxypropyl methylcellulose had a methoxyl content of about 18 percent, a hydroxypropoxyl content of about 27 percent and a Brookfield viscosity of about 59,000 mPa·s, measured as a 2 wt.% aqueous solution at 20°C, and is commercially available from The Dow Chemical Company under the Trademark METHOCEL J75M hypromellose.

### Treatment Group 3: K1M

The K1M diet for Treatment Group 3 was the same as the diet for Treatment Group 1, except that it contained 50 g of METHOCEL K1M hypromellose, which is commercially available from The Dow Chemical Company, has a methoxyl content of 19-24 percent, a hydroxypropoxyl content of 7-12 percent and an Ubbelohde viscosity of about 1,000 mPa·s, measured as a 2 wt.% aqueous solution at 20°C.

### Treatment Group 4: K500M

The K500M diet for Treatment Group 4 was the same as the diet for Treatment Group 1, except that it contained 50 g of METHOCEL K500M hypromellose, which is commercially available from The Dow Chemical Company, has a methoxyl content of 19-24 percent, a hydroxypropoxyl content of 7-12 percent and an Ubbelohde viscosity of about 500,000 mPa·s, measured as a 2 wt.% aqueous solution at 20°C.

### Control Group: MCC

The control diet had exactly the same composition as treatment diet, with the only exception that the hydroxypropyl methylcellulose derivative was replaced by same amount of microcrystalline cellulose (MCC), mixed into powdered components of diet during the control diet preparation.

### Analysis of Adiponectin in Hamster Plasma

Hamster EDTA plasma samples were assayed for adiponectin based on a double-antibody sandwich enzyme immunoassay technique. The adiponectin assay was conducted in a similar manner as described in Example 4.

### Analysis of PAI-1 in Hamster Plasma

Hamster EDTA plasma samples were assayed for Plasminogen Activator Inhibitor-1 (PAI-1) activity based on the inhibition of the plasminogen activator (urokinase (uPA) or tissue plasminogen activator (tPA)) activity of the synthetic chromogenic substrate method. The PAI-1 assay was conducted in a similar manner as described in Example 4.

After the hamster plasma was obtained from the different diets the plasma was analyzed for PAI-1 and adiponectin. The results are listed in Table 7.

**Table 7**

| Diet | [PAI] | Ratio | [Adiponectin] | Ratio |
|---|---|---|---|---|
| J1M | 4.8±0.8 | 1.02 | 10.4±1.8 | 1.19 |
| J75M | 4.9±0.8 | 1.05 | 10.8±2.6 | 1.23 |
| K1M | 4.6±0.9 | 0.99 | 9.4±2.3 | 1.07 |
| K500M | 4.6±0.8 | 0.98 | 11.1±2.2 | 1.27 |
| MCC | 4.7±0.9 | - | 8.8±1.6 | - |

| | | | | |
|---|---|---|---|---|
| *mean ± standard deviation | | | | |

The data show some variation; this is to be expected since the results are obtained on biological, living systems. Collectively, the results in Example 6 above illustrate the usefulness of a water-soluble cellulose derivative, such as hydroxypropyl methylcellulose, for influencing the level of expression or the concentration of adiponectin which is taught to be closely associated with the multiple risk factors that go to make up the metabolic syndrome.

### Example 7

Male Syrian golden hamsters of the same type and starting body weight as in Example 1 were divided into three groups, and each of these three groups was divided into three subgroups, labeled as Month 3, Month 8 and Month 10. The feeding period for Month 3 subgroups was 3 months, while it was 8 months for Month 8 subgroups and 10 months for Month 10 subgroups.

To prepare the diets of the Treatment group, a water-soluble cellulose ether was mixed with the powdered components of the diet. The water-soluble cellulose ether was hydroxypropyl methylcellulose (HPMC). The HPMC had a methoxyl content of 19-24 percent, a hydroxypropoxyl content of 7-12 percent and a viscosity of about 100,000 mPa·s, measured as a 2 wt.% aqueous solution at 20°C, and is commercially available from The Dow Chemical Company under the Trademark METHOCEL K100M hypromellose.

### Treatment Group: High Fat Diet, 5 weight percent HPMC

This treatment group was fed a high-fat treatment diet and water ad libitum. 1000 g of the high-fat treatment diet contained 150 g of butter fat, 50 g of corn oil, 200 g of casein, 499 g of corn starch, 3 g of DL methionine, 3 g of choline bitartrate, 35 g of a mineral mixture, 10 g of a vitamin mixture and 50 g of METHOCEL K100M hypromellose.

### High Fat Control Group:

The high-fat treatment diet for the High Fat Control Group was the same as the diet for the Treatment Group, except that it contained 50 g of microcrystalline cellulose (MCC) instead of 50 g of METHOCEL K100M hypromellose.

### Low Fat Control Group

The low-fat treatment diet contained most of the same ingredients at the same proportions as the High Fat Control Group, except that it did not contain 150 g of butterfat and it only contained 50 g of corn oil, thus it had 5% fat in general. The weight of butterfat (150 g) was replaced by the same amount of corn starch. Similar to the High Fat Control Group 1, the Low Fat Control Group contained 50 g of microcrystalline cellulose (MCC) instead of 50 g of METHOCEL K100M hypromellose.

The systolic blood pressure of the hamsters was measured for all groups and their subgroups, after feeding with the above-described experimental diet for the respective amount of time (i.e. Month 3 = 3 months; Month 8 = 8 months, Month 10 = 10 months). The subgroups aligned with the same period of time (i.e. 3, 8 or 10 months) were sacrificed after systolic blood pressure measurements were taken. The results are listed in Table 8 below. Month 3 subgroups averaged 6-8 animals per subgroup, with 3-5 repetitive measurements per animal. Month 8 subgroups averaged 11-12 animals per subgroup, with 3-5 repetitive measurements per animal. Month 10 subgroups averaged 4-8 animals per subgroup, with 3-5 repetitive measurements per animal. The values of the 3-5 repetitive measurements for each animal were subjected to a statistical outlier analysis using the Grubb's analysis [Grubbs, Frank (February 1969), Procedures for Detecting Outlying Observations in Samples, Technometrics, Vol. 11, No. 1, pp. 1-21. and http://www.itl.nist.gov/div898/handbook/eda/section3/eda35h.htm].

**Table 8**

| **Diet groups** | **Mean BP Systolic [mm Hg] and Standard Error of Mean (SEM)** | | | |
|---|---|---|---|---|
| **Subgroups*** | **Month 0** | **Month 3** | **Month 8** | **Month 10** |
| Treatment Group 5% | Not assessed | 83.78±3.48 | 86.67±5.12 | 76.92±4.15 |
| High Fat diet Control Group | Not assessed | 98.19±3.96 | 92.55±5.77 | 93.89±2.64 |
| Low fat diet Control Group | Not assessed | 87.97±3.03 | 86.71±4.65 | 90.00±1.56 |

| | | | | |
|---|---|---|---|---|
| * Each subgroup represents different animals, but on the same type of diet over different period of time - 3, 8 or 10 months, respectively | | | | |

## Claims

1. Hydroxypropyl methylcellulose for use in the treatment of metabolic syndrome.

2. Hydroxypropyl methylcellulose according to claim 1 for use in combination with ethyl cellulose in the treatment of metabolic syndrome.

3. Hydroxypropyl methylcellulose according to claim 1 for use in treating three or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

4. Hydroxypropyl methylcellulose according to claim 3 for use in combination with ethyl cellulose in treating three or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

5. Hydroxypropyl methylcellulose according to claim 1 or claim 3 for use in treating four or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

6. Hydroxypropyl methylcellulose according to claim 5 for use in combination with ethyl cellulose in treating four or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

7. A medicament, pharmaceutical composition, food, food ingredient or supplement, or nutraceutical ingredient or supplement comprising an effective amount of hydroxypropyl methylcellulose for use in treating metabolic syndrome.

8. A medicament, pharmaceutical composition, food, food ingredient or supplement, or nutraceutical ingredient or supplement of claim 7 comprising an effective amount of hydroxypropyl methylcellulose for use in combination with ethyl cellulose in treating metabolic syndrome.

9. The medicament, pharmaceutical composition, food, food ingredient or supplement, or nutraceutical ingredient or supplement of Claim 7 comprising an effective amount of hydroxypropyl methylcellulose for use in treating three or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

10. The medicament, pharmaceutical composition, food, food ingredient or supplement, or nutraceutical ingredient or supplement of Claim 9 comprising an effective amount of hydroxypropyl methylcellulose for use in combination with ethyl cellulose in treating three or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

11. The medicament, pharmaceutical composition, food, food ingredient or supplement, or nutraceutical ingredient or supplement of Claim 9 comprising an effective amount of hydroxypropyl methylcellulose for use in treating four or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

12. The medicament, pharmaceutical composition, food, food ingredient or supplement, or nutraceutical ingredient or supplement of Claim 11 comprising an effective amount of hydroxypropyl methylcellulose for use in combination with ethyl cellulose in treating four or more symptoms of the metabolic syndrome selected from the group consisting of i) hypertension, ii) proinflammatory or inflammation state, iii) prothrombotic state, iv) atherogenic dyslipidemia and v) insulin resistance with or without glucose intolerance.

## Patentansprüche

1. Hydroxypropylmethylcellulose zur Verwendung bei der Behandlung von metabolischem Syndrom.

2. Hydroxypropylmethylcellulose gemäß Anspruch 1 zur Verwendung in Kombination mit Ethylcellulose bei der Behandlung von metabolischem Syndrom.

3. Hydroxypropylmethylcellulose gemäß Anspruch 1 zur Verwendung beim Behandeln von drei oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

4. Hydroxypropylmethylcellulose gemäß Anspruch 3 zur Verwendung in Kombination mit Ethylcellulose beim Behandeln von drei oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

5. Hydroxypropylmethylcellulose gemäß Anspruch 1 oder Anspruch 3 zur Verwendung beim Behandeln von vier oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

6. Hydroxypropylmethylcellulose gemäß Anspruch 5 zur Verwendung in Kombination mit Ethylcellulose beim Behandeln von vier oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

7. Ein Medikament, eine pharmazeutische Zusammensetzung, ein Nahrungsmittel, ein Nahrungsmittelbestandteil oder eine Nahrungsmittelergänzung oder ein nutrazeutischer Bestandteil oder eine nutrazeutische Ergänzung, beinhaltend eine wirksame Menge Hydroxypropylmethylcellulose zur Verwendung beim Behandeln von metabolischem Syndrom.

8. Medikament, pharmazeutische Zusammensetzung, Nahrungsmittel, Nahrungsmittelbestandteil oder Nahrungsmittelergänzung oder nutrazeutischer Bestandteil oder nutrazeutische Ergänzung gemäß Anspruch 7, beinhaltend eine wirksame Menge Hydroxypropylmethylcellulose zur Verwendung in Kombination mit Ethylcellulose beim Behandeln von metabolischem Syndrom.

9. Medikament, pharmazeutische Zusammensetzung, Nahrungsmittel, Nahrungsmittelbestandteil oder Nahrungsmittelergänzung oder nutrazeutischer Bestandteil oder nutrazeutische Ergänzung gemäß Anspruch 7, beinhaltend eine wirksame Menge Hydroxypropylmethylcellulose zur Verwendung beim Behandeln von drei oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

10. Medikament, pharmazeutische Zusammensetzung, Nahrungsmittel, Nahrungsmittelbestandteil oder Nahrungsmittelergänzung oder nutrazeutischer Bestandteil oder nutrazeutische Ergänzung gemäß Anspruch 9, beinhaltend eine wirksame Menge Hydroxypropylmethylcellulose zur Verwendung in Kombination mit Ethylcellulose beim Behandeln von drei oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

11. Medikament, pharmazeutische Zusammensetzung, Nahrungsmittel, Nahrungsmittelbestandteil oder Nahrungsmittelergänzung oder nutrazeutischer Bestandteil oder nutrazeutische Ergänzung gemäß Anspruch 9, beinhaltend eine wirksame Menge Hydroxypropylmethylcellulose zur Verwendung beim Behandeln von vier oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

12. Medikament, pharmazeutische Zusammensetzung, Nahrungsmittel, Nahrungsmittelbestandteil oder Nahrungsmittelergänzung oder nutrazeutischer Bestandteil oder nutrazeutische Ergänzung gemäß Anspruch 11, beinhaltend eine wirksame Menge Hydroxypropylmethylcellulose zur Verwendung in Kombination mit Ethylcellulose beim Behandeln von vier oder mehr Symptomen des metabolischen Syndroms, ausgewählt aus der Gruppe, bestehend aus i) Hypertonie, ii) proinflammatorischem Zustand oder Entzündungszustand, iii) prothrombotischem Zustand, iv) atherogener Dyslipidämie und v) Insulinresistenz mit oder ohne Glucoseintoleranz.

## Revendications

1. Hydroxypropyl méthylcellulose destinée à être utilisée dans le traitement du syndrome métabolique.

2. Hydroxypropyl méthylcellulose selon la revendication 1 destinée à être utilisée en combinaison avec de l'éthylcellulose dans le traitement du syndrome métabolique.

3. Hydroxypropyl méthylcellulose selon la revendication 1 destinée à être utilisée dans le traitement de trois symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

4. Hydroxypropyl méthylcellulose selon la revendication 3 destinée à être utilisée en combinaison avec de l'éthylcellulose dans le traitement de trois symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

5. Hydroxypropyl méthylcellulose selon la revendication 1 ou la revendication 3 destinée à être utilisée dans le traitement de quatre symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

6. Hydroxypropyl méthylcellulose selon la revendication 5 destinée à être utilisée en combinaison avec de l'éthylcellulose dans le traitement de quatre symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

7. Un médicament, une composition pharmaceutique, un aliment, un ingrédient ou complément alimentaire, ou un ingrédient ou complément nutraceutique comprenant une quantité efficace d'hydroxypropyl méthylcellulose destinée à être utilisée dans le traitement du syndrome métabolique.

8. Un médicament, une composition pharmaceutique, un aliment, un ingrédient ou complément alimentaire, ou un ingrédient ou complément nutraceutique de la revendication 7 comprenant une quantité efficace d'hydroxypropyl méthylcellulose destinée à être utilisée en combinaison avec de l'éthylcellulose dans le traitement du syndrome métabolique.

9. Le médicament, la composition pharmaceutique, l'aliment, l'ingrédient ou le complément alimentaire, ou l'ingrédient ou le complément nutraceutique de la revendication 7 comprenant une quantité efficace d'hydroxypropyl méthylcellulose destinée à être utilisée dans le traitement de trois symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

10. Le médicament, la composition pharmaceutique, l'aliment, l'ingrédient ou le complément alimentaire, ou l'ingrédient ou le complément nutraceutique de la revendication 9 comprenant une quantité efficace d'hydroxypropyl méthylcellulose destinée à être utilisée en combinaison avec de l'éthylcellulose dans le traitement de trois symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

11. Le médicament, la composition pharmaceutique, l'aliment, l'ingrédient ou le complément alimentaire, ou l'ingrédient ou le complément nutraceutique de la revendication 9 comprenant une quantité efficace d'hydroxypropyl méthylcellulose destinée à être utilisée dans le traitement de quatre symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.

12. Le médicament, la composition pharmaceutique, l'aliment, l'ingrédient ou le complément alimentaire, ou l'ingrédient ou le complément nutraceutique de la revendication 11 comprenant une quantité efficace d'hydroxypropyl méthylcellulose destinée à être utilisée en combinaison avec de l'éthylcellulose dans le traitement de quatre symptômes ou plus du syndrome métabolique sélectionnés dans le groupe constitué i) de l'hypertension, ii) d'un état d'inflammation ou pro-inflammatoire, iii) d'un état prothrombotique, iv) de la dyslipidémie athérogène et v) de l'insulinorésistance avec ou sans intolérance au glucose.
